# FASCICULE DE BREVET EUROPEEN

(11) **EP 2 509 584 B1**
(45) Date de publication et mention de la délivrance du brevet: **19.02.2014**
(21) Numéro de dépôt: 10790436.9
(22) Date de dépôt: 10.12.2010
(51) Int. Cl.: A61K 9/107, A61K 36/00, A61P 17/02, A61K 35/64, A61K 31/19, A61K 31/21, A61K 45/06

(54) **COMPOSITION PHARMACEUTIQUE POUR LE TRAITEMENT DES BRÛLURES CUTANÉES**
PHARMAZEUTISCHE ZUSAMMENSETZUNG ZUR BEHANDLUNG VON HAUTVERBRENNUNGEN
PHARMACEUTICAL COMPOSITION FOR TREATING CUTANEOUS BURNS

(30) Priorité: 11.12.2009 BE 200900780
(43) Date de publication de la demande: 17.10.2012
(73) Titulaire: 45 Sec. LLC, New York, NY 10013 (US)
(72) Inventeur: Debetencourt, Jean-Jacques, Walmer, Kent CT14 7JX (GB)
(74) Mandataire: Office Freylinger
(86) Numéro de dépôt international: PCT/EP2010/069412
(87) Numéro de publication internationale: WO 2011/070168

(56) Documents cités:
- EP-A1- 0 870 507
- NL-A- 9 101 053
- US-A- 6 099 866
- US-A1- 2008 031 905

## Description

### Domaine technique

J La présente invention concerne le domaine des compositions pharmaceutiques utiles pour le traitement des lésions et des contractions cutanées dues à des brûlures thermiques ainsi que leur utilisation et les méthodes de traitement thérapeutiques associées.

### Etat de la technique

La brûlure cutanée, hors le cas de brûlure chimique, peut être définie comme une agression thermique de la peau. Une brûlure cutanée peut entraîner une destruction partielle ou totale de la peau, des tissus mous, des oreilles et yeux, des cheveux et poils, des ongles et même des os. La plupart des brûlures affectent uniquement la peau, à savoir : l'épiderme et le derme. Plusieurs facteurs sont pris en compte pour évaluer la gravité d'une brûlure, à savoir: la surface affectée, la profondeur, la localisation ainsi que la cause de la brûlure.

Les brûlures cutanées sont habituellement répertoriées selon leurs degrés de gravité. Une brûlure de premier degré se limite en général à un érythème et affecte uniquement les couches superficielles de l'épiderme. Une brûlure de deuxième degré «A» affecte l'épiderme ainsi qu'une partie du derme. Ces brûlures se manifestent par l'apparition d'un érythème ainsi que de phlyctènes à sa surface. Ce type de brûlure peut être à l'origine d'une douleur plus ou moins aiguë selon le niveau d'atteinte des nerfs. La rupture de capillarité sanguine peut déjà y être associée. Une brûlure de second degré « B » profonde peut s'étendre au-delà du derme et atteindre les tissus mous sub-cutanés. Une brûlure de troisième degré entraîne la destruction ou vitrification totale de l'épiderme et du derme. Ce type de brûlure endommage souvent les tissus sous-cutanés comme le tissu vasculaire, les muscles, et les nerfs. Pour traiter ce type de brûlure, il est d'usage de pratiquer des greffes de peau puisque aucune cicatrisation épidermique n'est théoriquement possible. La brûlure de quatrième degré affecte les muscles et peut même s'étendre jusqu'aux os. Dans ce cas, l'aspect de la peau est dit « carbonisé » et le traitement préconisé peut être l'amputation. Dans ce cas, le pronostic vital du patient est alors souvent engagé en fonction de l'âge du patient et de la surface atteinte.

Les brûlures peuvent être provoquées par différentes causes telles que :
- le contact avec une source de chaleur solide, liquide ou gazeuse (brûlure thermique),
- la radiation d'une source de chaleur (brûlure thermique),
- certains traitements médicaux tels que la radiothérapie,
- le contact avec le froid (une gelure),
- une électrocution (brûlure électrique),
- le contact avec un produit chimique (brûlure chimique),
- un frottement (brûlure de frottement qui est généralement assimilée à une brûlure thermique).

Selon l'Organisation Mondiale de la Santé, les brûlures seraient responsables de plus de 300000 décès annuels dans le monde (hors les cas de guerre). En France, en 2005, la Direction Générale de l'Offre de Soins (DGOS) dénombrait plus de 400000 cas de brûlures ayant nécessité des soins médicaux et dont 70% seraient liées à un accident domestique. Les brûlures nécessitant des soins de confort pourraient concerner 10% de la population. Il existe donc un réel besoin de proposer des compositions pharmaceutiques permettant de traiter les brûlures cutanées.

Des compositions pharmaceutiques destinées à traiter les brûlures sont déjà connues. Par exemple, le document NL9101053 décrit une émulsion huile-dans-eau formant un film continu presque non soluble et imperméable. Cette émulsion huile-dans-eau contient en outre du cétéaryl stéarate et est utile pour le traitement des peaux irritées et des brûlures cicatrisées.

Néanmoins, il est généralement observé que la durée de guérison totale d'une brûlure cutanée du premier ou du deuxième degré peut atteindre jusqu'à deux semaines.

Il apparaît donc nécessaire de proposer des compositions pharmaceutiques alternatives ainsi que des méthodes de traitement thérapeutiques pour le traitement des brûlures cutanées.

### Description générale de l'invention

La présente invention concerne une composition pharmaceutique comprenant au moins 4% en poids par rapport au poids total de la composition d'un principe actif choisi parmi le cétéaryl octanoate et/ou l'acide hexanoïque, en association avec de la cire d'abeille et optionnellement un ou plusieurs excipient(s) pharmaceutiquement acceptable(s) pour son utilisation dans le traitement des pré-brûlures ou brûlures cutanées.

Les recherches mises en oeuvre dans le cadre de la présente invention ont permis de mettre en évidence que lors du processus de formation d'une brûlure plusieurs étapes peuvent être identifiées :
- 1) contact avec la cause de la brûlure, (appelé également impact de chaleur),
- 2) début de la déformation (ou altération) de l'épiderme puis du derme, des tissus mous sub-cutanés et éventuellement du tissu vasculaire voire des organes, cette déformation s'assimile à un rétrécissement ou contraction des tissus,
- 3) poursuite de la radiation de la chaleur et de la déformation des différents tissus locaux ou avoisinants parallèlement à l'évaporation de l'eau contenue dans les tissus,
- 4) stabilisation des nécroses et des déformations des tissus (brûlures cutanées stabilisées qui peut prendre plusieurs mois pour les cas graves.

Au vu de ceci, il apparaît étonnant que les compositions pharmaceutiques déjà connues semblent traiter uniquement les lésions cutanées occasionnées par une brûlure après la stabilisation des nécroses et des déformations des tissus (étape 4). Une des raisons est certainement que la consultation chez le médecin a lieu lors de la phase de stabilisation (étape 4) et non lors de la dynamique de la transformation interne des tissus (étapes 2 et 3).

Néanmoins, il apparaît donc envisageable d'intervenir à différentes étapes du processus de formation de la brûlure (par exemple, aux étapes 2, 3 et/ou 4). Un des mérites de l'invention est d'avoir permis d'identifier un état particulier lors du processus de formation des brûlures cutanées à savoir, l'état de pré-brûlure correspondant aux étapes 2 et 3 précédemment décrites. Le terme pré-brûlure désigne au sens de la présente invention, les lésions cutanées correspondant à la déformation de l'épiderme, du derme et des tissus associée à l'évaporation de l'eau qui y est contenue; et qui sont occasionnées dans un délai généralement inférieur ou égal à 60 secondes et en particulier dans un délai inférieur ou égal à 45 secondes à compter du contact avec la cause de la brûlure.

Par conséquent, la composition pharmaceutique selon la présente invention se propose de traiter les lésions et déformations cutanées lorsqu'il s'agit encore de pré-brûlures (étapes 2 et 3 précédemment définies). Un autre mérite de l'invention est d'avoir mis en évidence que le traitement des lésions et déformations cutanées au stade pré-brûlure permettait une guérison totale plus rapide car intervenant avant la stabilisation. On croit que cette guérison est de 300% à 500% plus rapide dans la plupart des cas.

De plus, il a été mis en évidence que la composition pharmaceutique selon la présente invention était non seulement efficace pour le traitement des brûlures cutanées stabilisées (étape 4), par apport interne de certains éléments lipidiques, mais également et surtout pour le traitement des pré-brûlures (étapes 2 et 3).

De manière surprenante, il il a été constaté que l'utilisation de la composition pharmaceutique selon la présente invention permet de réduire nettement la durée de guérison totale d'une brûlure ou d'une pré-brûlure cutanée.

Sans vouloir être lié par une quelconque théorie, il semblerait que la déshydratation provoquée par l'impact de la source de chaleur est immédiatement compensée par l'application de la composition pharmaceutique selon l'invention. La composition selon l'invention permet une hydratation immédiate de la zone affectée pour restituer l'eau évaporée au sein des cellules et ainsi limiter l'afflux de sang ou de liquide physiologique au niveau de la zone affectée. De cette façon, la formation d'érythème ou de phlyctène est considérablement limitée. De plus, cette réhydratation rapide des tissus permettrait ainsi de réduire leur nécrose ou leur détérioration. Il semblerait également que la température plus élevée que présente les lésions cutanées observées au stade de la pré-brûlure par rapport à la température normale du corps permette une meilleure pénétration de la composition pharmaceutique. Ainsi, la durée de guérison totale d'une brûlure est significativement réduite. En outre, cette guérison rapide totale permet d'éviter les problèmes liés aux risques d'infection par des micro-organismes qui apparaissent souvent après 7 jours, en fonction de l'état de circulation sanguine locale. De plus, les terminaisons nerveuses étant moins comprimées, la douleur ressentie est réduite. Dans les cas non graves, elle disparaît après 10 minutes en raison de la non compression des nerfs par les tissus rétrécis. L'utilisation de la composition pharmaceutique selon la présente invention permet donc de diminuer les séquelles cutanées qui empêchent l'utilisation du membre brûlé et nécessitent des soins pour éviter les infections. De plus, l'utilisation de la composition selon la présente invention permet en outre de diminuer les cicatrices qui restent habituellement visibles pendant plusieurs mois.

De façon surprenante, il a été observé que la composition pharmaceutique selon la présente invention présente une pénétration cutanée rapide.

L'invention concerne l'utilisation de la composition pharmaceutique selon la présente invention pour le traitement des brûlures cutanées et en particulier pour le traitement des pré-brûlures cutanées. Un autre objet de la présente invention concerne une composition pharmaceutique caractérisée en ce qu'elle comprend au moins 4% en poids par rapport au poids total de la composition d'un principe actif choisi parmi le cétéaryl octanoate et/ou l'acide hexanoïque, en association avec de la cire d'abeille, un ou plusieurs additif(s) supplémentaire(s) choisi(s) parmi le dexpanthénol et/ou le polydiméthylsiloxane (diméthicone) et optionnellement un ou plusieurs excipient(s) pharmaceutiquement acceptable(s) ainsi que son utilisation en tant que médicament.

Il est également décrit dans le présent document une méthode thérapeutique caractérisée en ce que ladite composition pharmaceutique est mise en contact avec la brûlure cutanée et en particulier sur une pré-brûlure cutanée.

### Description du dessin

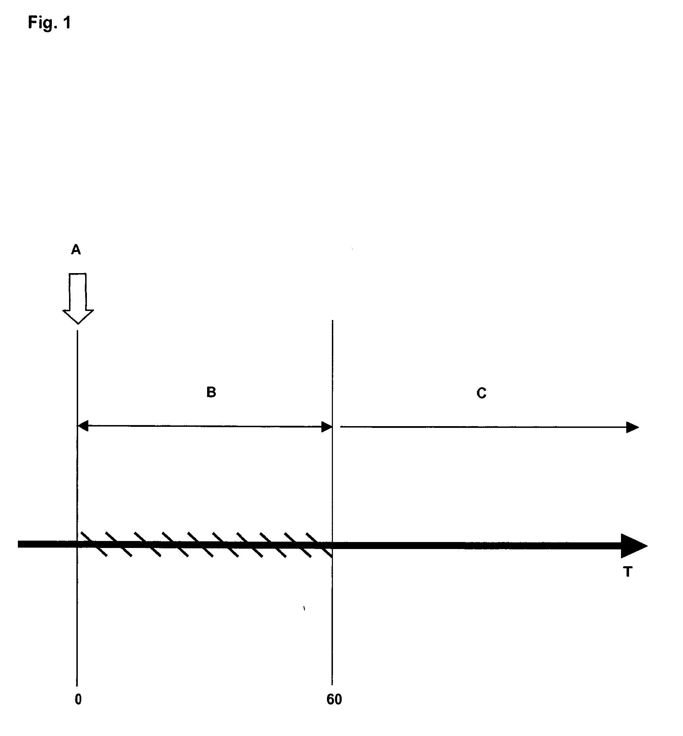

- Fig.1 : schéma illustrant le mode d'action de la composition pharmaceutique selon l'invention. (T) représente l'axe des temps en secondes. (A) représente le contact avec la cause de chaleur (ou impact de chaleur). (B) correspond à la période de pré-brûlure cutanée durant laquelle la composition selon l'invention agit de façon préférentielle et qui est comprise entre 0 et 60 secondes. (C) correspond à la période de stabilisation des nécroses et des déformations des tissus (brûlures stabilisée).

### Description détaillée de l'invention

La présente invention concerne une composition pharmaceutique caractérisée en ce qu'elle comprend au moins 4% en poids par rapport au poids total de la composition d'un principe actif choisi parmi le cétéaryl octanoate ou l'acide hexanoïque ou une combinaison des deux, en association avec de la cire d'abeille et optionnellement un ou plusieurs excipient(s) pharmaceutiquement acceptable(s) pour son utilisation dans le traitement des pré-brûlures ou brûlures cutanées.

Selon la présente invention, le terme cétéaryl octanoate désigne un ester octanoïque d'alcool cétylique et d'alcool stéarylique de formule brute C₂₄H₄₈O₂.

Selon la présente invention, le terme acide hexanoïque désigne le composé de formule générale C₆H₁₂O₂.

Selon la présente invention, le terme « cire d'abeille » désigne la cire d'abeille d'origine naturelle ou la cire d'abeille synthétique (CAS 71243-51-1) ou la cire d'abeille blanche.

Dans la présente invention, on entend désigner par « pharmaceutiquement acceptable » ce qui est utile dans la préparation d'une composition pharmaceutique, qui est généralement sûr, non toxique et ni biologiquement ni autrement non souhaitable et qui est acceptable pour une utilisation thérapeutique, notamment par application topique.

Avantageusement, la composition pharmaceutique selon l'invention comprend entre 4 à 15% en poids de principe actif par rapport au poids total de la composition. Selon un mode de réalisation de l'invention, la composition pharmaceutique contient au moins 4%, de préférence au moins 5%, de préférence au moins 6,5%, de préférence au moins 8%, de préférence au moins 10%, de préférence au moins 12% et de façon encore plus préférée au moins 14,5% en poids de principe actif par rapport au poids total de la composition. Selon un autre mode de réalisation de l'invention, la composition pharmaceutique contient au maximum 15%, de préférence au maximum 12%, de préférence au maximum 10%, de préférence au maximum 8%, de préférence au maximum 6,5% et de préférence au maximum 5% en poids de principe actif par rapport au poids total de la composition. Préférentiellement, la composition pharmaceutique comprend entre 4 à 12%, de préférence entre 5 à 10% et de préférence entre 6,5 à 8% en poids de principe actif par rapport au poids total de la composition.

De façon préférée, la composition pharmaceutique comprend entre 2 à 5% en poids de cire d'abeille par rapport au poids total de la composition pharmaceutique. Selon un mode de réalisation de l'invention, la composition pharmaceutique comprend au moins 2%, de préférence au moins 2,2%, de préférence au moins 2,5%, de préférence au moins 3%, de préférence au moins 3,5%, et selon un mode de réalisation au moins 4% en poids de cire d'abeille par rapport au poids total de la composition pharmaceutique. Selon un autre mode de réalisation de l'invention, la composition pharmaceutique comprend au maximum 5%, de préférence au maximum 4%, de préférence au maximum 3%, de préférence au moins 2,5% et de préférence au moins 2,2% en poids de cire d'abeille par rapport au poids total de la composition pharmaceutique. Avantageusement, la composition pharmaceutique comprend entre 2 à 5% et de préférence entre 2 à 4% en poids de cire d'abeille par rapport au poids total de la présente invention.

Avantageusement, le ratio entre le principe actif et le cire d'abeille est compris entre 0,8 et 7,5. Selon un mode de réalisation ledit ratio est compris entre 1,5 et 5 et de préférence est compris entre 2 et 4.

Selon un mode de réalisation de l'invention, le principe actif de la composition pharmaceutique est le cétéaryl octanoate.

De plus, la composition pharmaceutique peut en outre contenir un ou plusieurs additif(s) supplémentaire(s) choisi(s) parmi le dexpanthénol (vitamines B5) et/ou du polydiméthylsiloxane (diméthicone) dans le but notamment d'augmenter entre autres les propriétés hydratantes de la composition pharmaceutique.

Selon un mode de réalisation de l'invention, la composition pharmaceutique peut en outre comprendre du dexpanthénol. De façon préférée, la composition pharmaceutique comprend entre 0,1 et 5% en poids de dexpanthénol par rapport au poids total de la composition. Selon un mode de réalisation de l'invention, la composition pharmaceutique comprend au moins 0,1 %, de préférence au moins 0,2%, de préférence au moins 0,3 %, de préférence au moins 0,4%, de préférence au moins 1%, de préférence au moins 2%, de préférence au moins 3%, de préférence au moins 4% et de préférence au moins 4,5% en poids de dexpanthénol par rapport au poids total de la composition. Selon un autre mode de réalisation de l'invention, la composition pharmaceutique comprend au maximum 5%, de préférence au maximum 4%, de préférence au maximum 3%, de préférence au maximum 2%, de préférence au maximum 0,4%, de préférence au maximum 0,3% et de préférence au maximum 0,2% en poids de dexpanthénol par rapport au poids total de la composition. Avantageusement, la composition pharmaceutique comprend entre 0,2% et 3% en poids de dexpanthénol par rapport au poids total de la composition.

Selon un mode de réalisation de l'invention, la composition pharmaceutique peut en outre comprendre du polydiméthylsiloxane (diméthicone). De façon préférée, la composition pharmaceutique comprend entre 2 à 5% en poids de polydiméthylsiloxane par rapport au poids total de la composition pharmaceutique. Selon un mode de réalisation de l'invention, la composition pharmaceutique comprend au moins 2%, de préférence au moins 2,2%, de préférence au moins 2,5%, de préférence au moins 3%, de préférence au moins 4%, et selon un mode de réalisation au moins 4,5% en poids de polydiméthylsiloxane par rapport au poids total de la composition pharmaceutique. Selon un autre mode de réalisation de l'invention, la composition pharmaceutique comprend au maximum 5%, de préférence au maximum 4%, de préférence au maximum 3%, de préférence au maximum 2,5% et de préférence au maximum 2,2% en poids de polydiméthylsiloxane par rapport au poids total de la composition pharmaceutique. Avantageusement, la composition pharmaceutique comprend entre 2 à 4% et de préférence entre 2 à 3% en poids de polydiméthylsiloxane par rapport au poids total de la présente invention.

Selon un mode de réalisation de l'invention, la composition pharmaceutique comprend du cétéaryl octanoate, de la cire d'abeille, du dexpanthénol et du polydiméthylsiloxane.

La composition pharmaceutique peut comprendre en outre une ou plusieurs huile(s) essentielle(s) ou dérivés de celle(s)-ci, par exemple aux propriétés désinfectantes afin de prévenir une éventuelle infection d'origine bactérienne ou fongique ou aux propriétés apaisantes et hydratantes de la peau. Les huiles essentielles pourront éventuellement être raffinées. Les dérivés d'huile(s) essentielle(s) sont par exemple des extraits glycoliques, des teintures ou macérats d'huile(s) essentielle(s). Cette (ou ces) huile(s) essentielle(s) ou l'un de ses (leurs) dérivés pourra(ont) être sélectionnée(s) parmi le groupe constitué par : l'huile essentielle de Melaleuca Alterniflora ou l'un de ses dérivés, l'huile essentielle de Tepescohuite ou l'un de ses dérivés, l'huile essentielle d'eucalyptus ou l'un de ses dérivés, l'huile essentielle de thym ou l'un de ses dérivés, l'huile essentielle de romarin ou l'un de ses dérivés, l'huile essentielle de citron ou l'un de ses dérivés, l'huile essentielle de clou de girofle ou l'un de ses dérivés, l'huile essentielle de cannelle ou l'un de ses dérivés, l'huile essentielle de pin ou l'un de ses dérivés, l'huile essentielle de lavande ou l'un de ses dérivés, l'huile essentielle de cistus ladaniferus ou l'un de ses dérivés, l'huile essentielle de Millepertuis ou l'un de ses dérivés et l'huile essentielle de rosier muscat ou l'un de ses dérivés.

De façon préférée, l'huile ou les huiles essentielle(s) ou l'un de ses (leurs) dérivés sera sélectionnée(s) parmi le groupe constitué par l'huile essentielle de Melaleuca Alterniflora ou l'un de ses dérivés, l'huile essentielle de Tepescohuite ou l'un de ses dérivés, l'huile essentielle de Millepertuis ou l'un de ses dérivés et l'huile essentielle de rosier muscat ou l'un de ses dérivés.

En ce qui concerne les dérivés d'huile(s) essentielle(s), on peut en particulier citer l'extrait glycolique ou la teinture ou le macérat de Tepescohuite. On peut également citer l'extrait glycolique ou la teinture ou le macérat de Millepertuis.

Selon un mode de réalisation de l'invention, la composition pharmaceutique comprend entre 0,01% et 10% en poids d'huile(s) essentielle(s) ou l'un de ses (leurs) dérivés par rapport au poids total de la composition et de préférence entre 0,1% et 5% en poids d'huile(s) essentielle(s) ou l'un de ses (leurs) dérivés par rapport au poids total de la composition.

La composition pharmaceutique peut en outre contenir un ou plusieurs anesthésique(s) local(aux) choisi(s) parmi le groupe suivant : la lidocaïne, la prilocaïne, la scandicaïne, la prilocaïne, l'étidocaïne, la bupivacaïne, la ropivacaïne, la lévobupivacaïne, l'articaïne et la mépivacaïne. Selon un mode de réalisation de l'invention, il s'agira de la lidocaïne. De façon préférée, la composition pharmaceutique comprend entre 10 à 20% en poids d'anesthésique(s) local(aux) par rapport au poids total de la composition pharmaceutique. Selon un mode de réalisation, la composition pharmaceutique au moins 10%, et de préférence au moins 15% et de préférence au moins 18% en poids d'anesthésique(s) local(aux) par rapport au poids total de la composition pharmaceutique. Selon un autre mode de réalisation, la composition pharmaceutique comprend au maximum 20%, de préférence au maximum, 15% et de préférence au maximum 12% en poids d'anesthésique(s) local(aux) par rapport au poids total de la composition pharmaceutique. Avantageusement, la composition pharmaceutique comprend entre 10 à 15% en poids d'anesthésique(s) local(aux) par rapport au poids total de la composition pharmaceutique.

Selon un mode de réalisation, la composition pharmaceutique peut en outre comprendre de l'allantoïne.

La composition pharmaceutique peut se présenter sous les formes qui sont habituellement connues pour une administration topique, c'est à dire notamment les lotions, les mousses, les gels, les dispersions, les émulsions eau-dans-huile ou huile-dans-eau ou multiple, les sprays, les sérums, les masques, les laits corporels ou les crèmes, avec des excipients permettant notamment une pénétration cutanée afin d'améliorer les propriétés et l'accessibilité des principes actifs. La composition pharmaceutique peut se présenter sous les formes qui sont habituellement connues pour une administration par injection. Par injection, on entend une administration par voie sous-cutanée ou voie intradermique. Dans ce cas, la composition pharmaceutique se présente sous une forme injectable incorporée par exemple dans une seringue stérile. La stérilisation de la seringue pourra par exemple être réalisée par un traitement aux rayons bêta ou gamma.

Avantageusement, la composition pharmaceutique est une émulsion eau-dans-huile ou une émulsion huile-dans-eau.

La composition pharmaceutique peut en outre comprendre des excipients utilisés habituellement dans le domaine des compositions pharmaceutiques destinées à être appliquées par voie topique (par exemple compositions dermatologiques) ou par injection.

La phase grasse de la présente composition pharmaceutique peut en outre comprendre des composés lipophiles comme les huiles, les gommes, les pâtes et les cires.

Les huiles sont choisies de préférence parmi les huiles végétales, les huiles animales, les huiles minérales, les huiles de synthèse, les huiles siliconées, les esters d'acides gras liquides, les acides gras liquides et les amides gras liquides.

Comme huile végétale, on peut notamment mentionner l'huile d'amande douce (Prunus Amigdalus Dulcis), l'huile d'avocat, l'huile de ricin, l'huile d'olive, la cire liquide jojoba, l'huile de tournesol, l'huile de germes de blé, l'huile de sésame, l'huile d'arachide, l'huile de pépins de raisin, l'huile de soja, l'huile de colza, l'huile de cartham, l'huile de coprah, l'huile de maïs, l'huile de noisette, l'huile de noyau d'abricot et l'huile de calophyllum.

Comme huile animale, on peut notamment citer le perhydrosqualène.

Comme huile de synthèse, on peut citer le squalane, les poly(α-oléfines) comme l'isododécane ou l'isohexadécane, les huiles végétales transestérifiées et les huiles fluorées.

Comme huiles siliconées, on peut citer les polydiméthylsiloxanes cycliques (par exemple: cyclométhicone ou diméthicone) tels que le décaméthyl pentasiloxane et les polyméthylsiloxanes linéaires de faible viscosité (150 à 500cs).

En plus de la cire d'abeille utilisée dans la composition pharmaceutique peut éventuellement contenir les cires utilisables sont par exemple les cires d'origine animale, végétale, minérale ou synthétique telle que, les cires fluorées ou perfluorées, les cires de lanoline, les cires de Candellila, de beurre de cacao, de beurre de karité, la cire de sapin, la cire de coton ; les cires microcristallines, la cire de paraffine, le petrolatum, la vaseline, l'ozokérite; les huiles hydrogénées ayant une température de fusion supérieure à 40°C comme l'huile de jojoba hydrogénée, les cires de polyéthylène.

La composition pharmaceutique peut en outre contenir des émulsionnants habituellement utilisés. Le système émulsionnant peut comprendre en particulier un ou plusieurs composés choisis parmi les alcools gras éthoxylés, les esters d'acides gras et de PEG, les glycérides partiels d'acides gras éthoxylés, les triglycérides d'acide gras polyglycérolés et leurs dérivés éthoxylés. On peut citer en tant qu'alcools gras éthoxylés appropriés, les produits d'addition d'oxyde d'éthylène avec l'alcool béhénylique, notamment ceux comportant de 6 à 12 groupes oxyéthylénés (par exemple Beheneth-9 ou Beheneth-10) ; les produits d'additions d'oxyde d'éthylène avec l'alcool stéarylique, notamment ceux comportant 6 à 12 groupes oxyéthylénés (par exemple steareth-9 ) ; les produits d'addition d'oxyde d'éthylène avec l'alcool isostéarylique, par exemple ceux comportant 6 à 12 groupes oxyéthylénés (Isosteareth-9), et leurs mélanges.

La composition pharmaceutique peut comprendre des tensio-actifs non ioniques, des alcools gras oxyéthylénés différents de ceux décrits précédemment, à savoir les produits d'addition d'oxyde d'éthylène avec l'alcool laurylique (laureth-9 à laureth-50) ; les produits d'addition d'oxyde d'éthylène avec l'alcool cétéarylique ou cétylstéarylique (Ceteareth-9 à Ceteareth-30), les produits d'addition d'oxyde d'éthylène avec l'alcool cétylique (Ceteth-9 à Ceteth-30) ; et leurs mélanges.

Des tensioactifs additionnels peuvent éventuellement être compris dans la composition. Ces tensioactifs additionnels peuvent être les sels d'acide gras ayant 8 à 30 atomes de carbones comme par exemple les sels d'acide palmitique, l'acide stéarique, l'acide béhénique, les esters gras de glycérol, comme par exemple le glycéryl stéarate ; les dérivés oxyéthylénés des sels d'acides gras et des esters gras de glycérol comportant 2 à 8 groupes d'oxyde d'éthylène et leurs mélanges.

La composition pharmaceutique peut en outre comprendre des conservateurs habituellement utilisés dans le domaine des compositions pharmaceutiques. Il peut s'agir notamment d'agents antimicrobiens tels que des conservateurs ou agents antifongiques choisis parmi les alcools, pouvant contenir un ou plusieurs substituants aromatiques, par exemple les phénoxyéthanol comme le 2-phénoxyéthanol, 1-phénoxy-2-propanol, l'alcool benzylique, le 2-hydroxybiphényl, les parabènes, comme le méthylparabène, éthylparabène, propylparabène, butylparabène, isobutylparabène, méthylparabène de sodium, éthylparabène de sodium, propylparabène de sodium, isobutylparabène de sodium, butylparabène de sodium ou isobutylparabène de sodium, le parahydroxybenzoate d'éthyl , de buthyl, de propyl, de méthyl, l'urée de l'imidazolidinyle, l'urée du diazolidinyle, l'hydroxyméthylglycinate de sodium, des dérivés halogénés tels que butylcarbamate d'iodopropynyle, le 2-bromo-2-nitropropan-1,3-diol, 2,4,4'-trichloro-2'-hydroxydiphényléther (triclosan), le 3,4,4'-trichlorocarbanilide (triclocarban), le chlorbutanulum, l'alcool 2,4-dichlorobenzylique, l'urée du N-(4-chlorphényl-N'-(3,4-dichlorphényle, le 1,2-dibromo-2,4-dicyanobutane, le chloroxylénol, le cétoconazole, l'oxiconazole, le butoconazole, le clotrimazole, l'éconazole, l'énilconazole, le fenticonazole, le miconazole, le sulconazole, le tioconazole, le fluconazole, l'itraconazole, le terconazole, des actifs contenant un ou plusieurs azotes cationiques tels que le chlorure de cétyltriméthylammonium, le chlorure de cétylpyridinium, le chlorure de benzéthonium, le chlorure de benzalkonium, éventuellement en association avec du peroxide d'hydrogène stabilisé au perborate, le chlorure de diisobutyléthoxy-éthyl-diméthylbenzylammonium, le chlorure de diisobutyl-phénoxy-éthoxyéthyl-diméthylbenzyl-ammonium, le chlorure, bromure, saccharinate de N-alkyl-N,N-diméthyl-benzyl-ammonium, le chlorure de triméthylammonium, l'aluminiumchloro-hydroxylacétate de sodium, le chlorure de tricétylméthylammonium, diaminoalkyl-amide, des acides organiques et leurs sels, tel que l'acide citrique, des agents antimicrobiens insaturés tels que le farnesol, la terbinafine ou la naftifine, des agents aromatiques hétérocycliques tels que le bifonazole, le cloconazole, l'isoconazole, de tout autre agent antimicrobien ou antifongique connu de l'homme du métier; et leurs mélanges.

En outre, la composition pharmaceutique peut également comprendre des acides et des bases permettant d'ajuster la zone de pH de ladite composition pharmaceutique. Les bases peuvent être minérales (soude, potasse, ammoniaque) ou organiques telles que la mono-, di- ou triéthanolamine, un aminométhylpropanediol, la N-méthyl-glucamine, tes acides aminés basiques comme l'arginine et la lysine; et leurs mélanges.

La présente invention concerne l'utitisation d'une composition pharmaceutique telle que définie précédemment pour le traitement des brûlures cutanées et en particulier pour le traitement des pré-brûlures cutanées.

La composition pharmaceutique selon la présente invention est utile pour traiter des pré-brûlures ou des brûlures cutanées d'origine différente, à savoir : les pré-brûlures ou les brûlures cutanées provoquées par contact ou radiation d'une source chaude, les pré-brûlures ou les brûlures cutanées provoquées par les traitements médicaux comme la radiothérapie, les pré-brûlures ou les brûlures cutanées provoquées par le contact avec le froid, les pré-brûlures ou les brûlures cutanées provoquées par un frottement, ainsi que les pré-brûlures ou les brûlures cutanées causées par une exposition de courte durée au soleil. Avantageusement, la composition pharmaceutique selon l'invention est en particulier utile pour traiter les pré-brûlures ou brûlures cutanées provoquées par contact ou radiation d'une source chaude, provoquées par les traitements médicaux comme la radiothérapie ou provoquées par un frottement. Selon un autre aspect de l'invention, la composition pharmaceutique peut être utile pour le traitement des pré-brûlures ou des brûlures des muqueuses (par exemple les parois du système digestif telles que l'oesophage ou les lèvres, les zones urogénitales, etc.). La composition pharmaceutique selon l'invention est utile pour traiter les pré-brûlures ou les brûlures du premier, deuxième degré ou du troisième degré.

Selon un aspect de l'invention, l'utilisation est caractérisée en ce que la composition pharmaceutique est mise en contact avec les lésions cutanées dans un délai inférieur ou égal à 60 secondes à compter du contact avec la cause de la pré-brûlure ou brûlure dans le cas d'une administration par voie topique ou dans un délai inférieur ou égal à 30 minutes à compter du contact avec la cause de la pré-brûlure ou brûlure dans le cas d'une administration par injection.

De façon préférée, l'utilisation est caractérisée en ce que la composition pharmaceutique est mise en contact avec les lésions cutanées dans un délai inférieur ou égal à 45 secondes à compter du contact avec la cause de la pré-brûlure ou brûlure dans le cas d'une administration par voie topique.

Selon un mode de réalisation de l'invention, l'utilisation est caractérisée en ce que la composition pharmaceutique est mise en contact avec les lésions cutanées dans un délai inférieur ou égal à 25 minutes à compter du contact avec la cause de la pré-brûlure ou brûlure dans le cas d'une administration par injection.

Selon un mode de réalisation de l'invention, l'utilisation de la composition pharmaceutique est caractérisée en ce que la composition pharmaceutique est administrée 30 fois de façon subséquente pendant 60 minutes, ou 20 fois pendant 60 minutes, ou 10 fois pendant 30 minutes, ou 5 fois pendant 15 minutes.

Le présent document décrit également une méthode de traitement thérapeutique caractérisée en ce que la composition pharmaceutique selon la présente invention est mise en contact avec une brûlure cutanée et en particulier avec une pré-brûlure cutanée.

Avantageusement, la méthode de traitement thérapeutique décrite est caractérisée en ce que la composition pharmaceutique est mise en contact avec les lésions cutanées dans un délai inférieur ou égal à 60 secondes à compter du contact avec la cause de la pré-brûlure ou brûlure cutanée dans le cas d'une administration par voie topique ou dans un délai inférieur ou égal à 30 minutes dans le cas d'une administration par injection.

De façon préférée, la méthode de traitement thérapeutique décrite est caractérisée en ce que la composition pharmaceutique est mise en contact avec les lésions cutanées dans un délai inférieur ou égal à 45 secondes à compter du contact avec la cause de la pré-brûlure ou brûlure cutanée dans le cas d'une administration par voie topique.

Selon un mode de réalisation, la méthode de traitement thérapeutique décrite est caractérisée en ce que la composition pharmaceutique est mise en contact avec les lésions cutanées dans un délai inférieur ou égal à 25 minutes à compter du contact avec la cause de la pré-brûlure ou brûlure cutanée dans le cas d'une administration par injection.

Le présent document décrit également une méthode de traitement thérapeutique pour réduire l'intensité de la douleur due à un choc thermique caractérisée en ce que la composition pharmaceutique est mise en contact avec les lésions cutanées dans un délai inférieur ou égal à 60 secondes à compter du contact avec la cause de la pré-brûlure ou brûlure cutanée dans le cas d'une administration par voie topique ou dans un délai inférieur ou égal à 30 minutes dans le cas d'une administration par injection. Un objet de la présente invention concerne une composition pharmaceutique caractérisée en ce qu'elle comprend au moins 4% en poids par rapport au poids total de la composition d'un principe actif choisi parmi le cétéaryl octanoate et/ou l'acide hexanoïque, en association avec de la cire d'abeille, un ou plusieurs additif(s) supplémentaire(s) choisi(s) parmi le dexpanthénol et/ou le polydiméthylsiloxane (diméthicone) et optionnellement un ou plusieurs excipient(s) pharmaceutiquement acceptable(s) ainsi que son utilisation en tant que médicament.

### - Exemple 1 : Essais cliniques

### - a) Protocole des essais cliniques

La composition pharmaceutique selon l'invention a été testée sur plusieurs patients présentant des pré-brûlures d'origine et de gravité différentes. Pour chacun des cas suivants, la composition pharmaceutique a été appliquée directement sur la pré-brûlure cutanée dans un délai de 45 secondes à compter du contact avec la cause de la brûlure puis de façon régulière pendant une période définie.

### - b) Résultats des essais cliniques

- Patient 1 : Ce patient présentait une pré-brûlure du deuxième degré dont l'origine provenait d'un contact direct du bras avec une résistance de four chauffée à 350°C. La composition pharmaceutique selon l'invention a été appliquée dans un délai de 45 secondes à compter du contact avec la résistance de four chauffée à 350°C, puis elle a été appliquée toutes les 3 minutes pendant 15 minutes puis de façon régulière pendant deux jours. A l'issue de cette période, toute trace visible des lésions cutanées a disparu : la zone de la peau affectée ne présentait pas d'érythèmes ni de phlyctènes et n'était pas décollée. La douleur a disparu après 3 heures.
- Patient 2 : Ce patient présentait une pré-brûlure du deuxième degré provoquée par le contact de la paume de la main avec une mèche de perçage rougie à 400°C. La composition pharmaceutique selon l'invention a été appliquée dans un délai de 45 secondes à compter du contact avec la mèche de perçage rougie, puis elle a été appliquée de façon régulière pendant deux jours. A l'issue de cette période, toute trace visible des lésions cutanées a disparu : la zone de la peau affectée ne présentait pas d'érythèmes ni de phlyctènes et n'était pas décollée. La douleur a disparu après 2 heures.
- Patient 3: Ce patient présentait une pré-brûlure du premier degré provoquée par contact de la main avec de la vapeur d'eau. La composition pharmaceutique selon l'invention a été appliquée dans un délai de 45 secondes à compter du contact avec la vapeur d'eau, puis elle a été appliquée de façon régulière pendant 3 heures. A l'issue de cette période, toute trace visible des lésions cutanées a disparu : la zone de la peau affectée ne présentait pas d'érythèmes ni de phlyctènes et n'était pas décollée. Ce cas a été répété sur 5 autres patients. La douleur a disparu après 10 minutes.
- Patient 4 : Ce patient présentait une pré-brûlure de 2^{nd} degré « A » provoquée par le frottement de la chaîne de sa tronçonneuse thermique sur la cuisse. La composition pharmaceutique selon l'invention a été appliquée dans un délai de 45 secondes à compter du contact avec la chaîne de tronçonneuse, puis elle a été appliquée de façon régulière pendant trois jours. A l'issue de cette période, toute trace visible de des lésions cutanées a disparu : la zone de la peau affectée ne présentait pas d'érythèmes ni de phlyctènes et n'était pas décollée. La douleur a disparu après 10 heures.
- Patient 5 : Ce patient présentait une pré-brûlure de 2^{nd} degré « B » provoquée par le contact d'un tuyau d'échappement de moto sur la jambe. La composition pharmaceutique selon l'invention a été appliquée dans un délai de 45 secondes à compter du contact avec le tuyau d'échappement de moto, puis elle a été appliquée de façon régulière pendant trois jours. A l'issue de 5 jours, toute trace visible des lésions cutanées a disparu : la zone de la peau affectée ne présentait pas d'érythèmes ni de phlyctènes et n'était pas décollée. La douleur a disparu après 24 heures.

Ces résultats démontrent que la composition pharmaceutique selon l'invention est particulièrement efficace pour traiter les pré-brûlures cutanées et réduire de façon significative les lésions cutanées qu'elles occasionnent. Alors qu'habituellement, il est observé que la durée nécessaire pour que les lésions cutanées occasionnées par une brûlure de deuxième degré disparaissent est d'environ deux semaines, dans le cas de l'utilisation de la composition pharmaceutique selon l'invention, cette durée est réduite à 2 ou 3 jours. Cette diminution significative de la durée de guérison permet en outre de réduire le risque d'infection provoquée par des micro-organismes (d'origine bactérienne ou fongique) souvent observée après 7 jours dans le cas de lésions cutanées provoquées par une brûlure. De plus, il est à noter que la douleur occasionnée par les lésions cutanées est significativement réduite par l'utilisation de la composition pharmaceutique selon l'invention.

### - Exemple 2 : Composition pharmaceutique pour son utilisation dans le traitement des pré-brûlures ou brûlures cutanées selon l'invention.

| **Quantité en %** | **Désignation** | **Fonction** |
|---|---|---|
| QSP 100% | Eau | Hydratation |
| 5-9% | Phase grasse et tensioactifs | Emulsionneurs |
| 2-5% | Polydiméthylsiloxane | Hydratation |
| 2-5% | Cire d'abeille | Agent nourrissant et apaisant |
| 4-15% | Cétéaryl éthylhexanoate | Principe actif |
| 3-5% | Sorbitol | Agent de phase aqueuse |
| 0,1-5% | Dexpanthénol | Hydratation |
| 1-1,5% | Alcool cétylique | Emulsionnant |
| 0,15-0,35% | Carbomère | Viscosant |
| 0,4-3% | Huile essentielle de Melaleuca alterniflora | Désinfectant |
| 0,1-0,9% | Huiles essentielles et macérats | Agent apaisant |
| 0,15% | Parabènes ou assimilés | Conservateurs |

## Revendications

1. Composition pharmaceutique **caractérisée en ce qu'**elle comprend au moins 4% en poids par rapport au poids total de la composition d'un principe actif choisi parmi le cétéaryl octanoate et/ou l'acide hexanoïque, en association avec de la cire d'abeille et optionnellement un ou plusieurs excipient(s) pharmaceutiquement acceptable(s) pour son utilisation dans le traitement des pré-brûlures ou brûlures cutanées.

2. Composition pharmaceutique pour une utilisation selon la revendication 1 **caractérisée en ce que** le principe actif est le cétéaryl octanoate.

3. Composition pharmaceutique pour une utilisation selon la revendication 1 ou 2 **caractérisée en ce qu'**elle comprend entre 4 à 15% en poids de principe actif par rapport au poids total de la composition.

4. Composition pharmaceutique pour une utilisation selon l'une des revendications 1 à 3 **caractérisée en ce qu'**elle comprend 2 à 5% en poids de cire d'abeille par rapport au poids total de la composition.

5. Composition pharmaceutique pour une utilisation selon l'une des revendications 1 à 4 **caractérisée en ce que** le ratio entre le principe actif et la cire d'abeille est compris entre 0,8 et 7,5.

6. Composition pharmaceutique pour une utilisation selon l'une des revendications 1 à 5 **caractérisée en ce qu'**elle comprend un ou plusieurs additif(s) supplémentaire(s) choisi(s) parmi le dexpanthénol et/ou le polydiméthylsiloxane (diméthicone).

7. Composition pharmaceutique pour une utilisation selon l'une des revendications 1 à 6 **caractérisée en ce qu'**elle comprend un ou plusieurs anesthésiques local(ux) choisi(s) parmi : la lidocaïne, la prilocaïne, la scandicaïne, la prilocaïne, l'étidocaïne, la bupivacaïne, la ropivacaïne, la lévobupivacaïne, l'articaïne et la mépivacaïne.

8. Composition pharmaceutique pour une utilisation selon l'une des revendications 1 à 7 **caractérisée en ce qu'**elle comprend en outre une ou plusieurs huile(s) essentielle(s) ou l'un de ses (leurs) dérivés sélectionnée(s) parmi le groupe constitué par : l'huile essentielle de Melaleuca Alterniflora ou l'un de ses dérivés, l'huile essentielle de Tepescohuite ou l'un de ses dérivés, l'huile essentielle d'eucalyptus ou l'un de ses dérivés, l'huile essentielle de thym ou l'un de ses dérivés, l'huile essentielle de romarin ou l'un de ses dérivés, l'huile essentielle de citron ou l'un de ses dérivés, l'huile essentielle de clou de girofle ou l'un de ses dérivés, l'huile essentielle de cannelle ou l'un de ses dérivés, l'huile essentielle de pin ou l'un de ses dérivés, l'huile essentielle de lavande ou l'un de ses dérivés, l'huile essentielle de cistus ladaniferus ou l'un de ses dérivés, l'huile essentielle de Millepertuis ou l'un de ses dérivés et l'huile essentielle de rosier muscat ou l'un de ses dérivés.

9. Composition pharmaceutique pour une utilisation selon l'une des revendications 1 à 8 **caractérisée en ce qu'**elle est administrée par voie topique ou par injection.

10. Composition pharmaceutique pour une utilisation selon l'une quelconque des revendications 1 à 9 **caractérisée en ce que** la composition pharmaceutique est mise en contact avec les lésions cutanées, dans un délai inférieur ou égal à 60 secondes à compter du contact avec la cause de la pré-brûlure ou brûlure dans le cas d'une administration par voie topique ou dans un délai inférieur ou égal à 30 minutes à compter du contact avec la cause de la pré-brûlure ou brûlure dans le cas d'une administration par injection.

11. Composition pharmaceutique pour une utilisation selon l'une des revendications 1 à 10 **caractérisée en ce que** la composition pharmaceutique est mise en contact avec les lésions cutanées dans un délai inférieur ou égal à 45 secondes à compter du contact avec la cause de la pré-brûlure ou brûlure dans le cas d'une administration par voie topique.

12. Composition pharmaceutique pour une utilisation selon l'une des revendications 1 à 10 **caractérisée en ce que** la composition pharmaceutique est mise en contact avec les lésions cutanées dans un délai inférieur ou égal à 25 minutes à compter du contact avec la cause de la pré-brûlure ou brûlure dans le cas d'une administration par injection.

13. Composition pharmaceutique pour une utilisation selon l'une des revendications 1 à 12 **caractérisée en ce que** la composition pharmaceutique est administrée 30 fois de façon subséquente pendant 60 minutes.

14. Composition pharmaceutique **caractérisée en ce qu'**elle comprend au moins 4% en poids par rapport au poids total de la composition d'un principe actif choisi parmi le cétéaryl octanoate et/ou l'acide hexanoïque, en association avec de la cire d'abeille, un ou plusieurs additif(s) supplémentaire(s) choisi(s) parmi le dexpanthénol et/ou le polydiméthylsiloxane (diméthicone) et optionnellement un ou plusieurs excipient(s) pharmaceutiquement acceptable(s).

15. Composition pharmaceutique selon la revendication 14 pour son utilisation en tant que médicament.

## Patentansprüche

1. Pharmazeutische Zusammensetzung, **dadurch gekennzeichnet, dass** sie mindestens 4 Gew.-%, bezogen auf das Gesamtgewicht der Zusammensetzung, eines Wirkstoffs, der aus Cetearyloctanoat und/oder Capronsäure ausgewählt ist, in Verbindung mit Bienenwachs und gegebenenfalls einem oder mehreren pharmazeutisch unbedenklichen Hilfsstoffen für deren Verwendung bei der Behandlung von Vor-Verbrennungen oder Verbrennungen der Haut umfasst.

2. Pharmazeutische Zusammensetzung für eine Verwendung nach Anspruch 1, **dadurch gekennzeichnet, dass** der Wirkstoff Cetearyloctanoat ist.

3. Pharmazeutische Zusammensetzung für eine Verwendung nach Anspruch 1 oder 2, **dadurch gekennzeichnet, dass** sie zwischen 4 und 15 Gew.-% Wirkstoff umfasst, bezogen auf das Gesamtgewicht der Zusammensetzung.

4. Pharmazeutische Zusammensetzung für eine Verwendung nach einem der Ansprüche 1 bis 3, **dadurch gekennzeichnet, dass** sie 2 bis 5 Gew.-% Bienenwachs umfasst, bezogen auf das Gesamtgewicht der Zusammensetzung.

5. Pharmazeutische Zusammensetzung für eine Verwendung nach einem der Ansprüche 1 bis 4, **dadurch gekennzeichnet, dass** das Verhältnis zwischen dem Wirkstoff und dem Bienenwachs zwischen 0,8 und 7,5 liegt.

6. Pharmazeutische Zusammensetzung für eine Verwendung nach einem der Ansprüche 1 bis 5, **dadurch gekennzeichnet, dass** sie ein oder mehrere zusätzliche Additive umfasst, der bzw. die aus Dexpanthenol und/oder Polydimethylsiloxan (Dimethicon) ausgewählt ist bzw. sind.

7. Pharmazeutische Zusammensetzung für eine Verwendung nach einem der Ansprüche 1 bis 6, **dadurch gekennzeichnet, dass** sie ein oder mehrere Lokalanästhetika umfasst, das bzw. die aus folgenden ausgewählt ist bzw. sind: Lidocain, Prilocain, Scandicain, Prilocain, Etidocain, Bupivacain, Ropivacain, Levobupivacain, Articain und Mepivacain.

8. Pharmazeutische Zusammensetzung für eine Verwendung nach einem der Ansprüche 1 bis 7, **dadurch gekennzeichnet, dass** sie außerdem ein oder mehrere ätherische Öle oder eines seiner bzw. ihrer Derivate umfasst, das bzw. die aus der Gruppe ausgewählt ist bzw. sind, die aus folgenden besteht: ätherisches Melaleuca-alterniflora-Öl oder eines seiner Derivate, ätherisches Tepezcohuite-Öl oder eines seiner Derivate, ätherisches Eukalyptusöl oder eines seiner Derivate, ätherisches Thymianöl oder eines seiner Derivate, ätherisches Rosmarinöl oder eines seiner Derivate, ätherisches Zitronenöl oder eines seiner Derivate, ätherisches Gewürznelkenöl oder eines seiner Derivate, ätherisches Zimtöl oder eines seiner Derivate, ätherisches Pinienöl oder eines seiner Derivate, ätherisches Lavendelöl oder eines seiner Derivate, ätherisches Cistus-ladaniferus-Öl oder eines seiner Derivate, ätherisches Johanniskrautöl oder eines seiner Derivate und ätherisches Muskatrosenöl oder eines seiner Derivate.

9. Pharmazeutische Zusammensetzung für eine Verwendung nach einem der Ansprüche 1 bis 8, **dadurch gekennzeichnet, dass** sie topisch oder mittels Injektion verabreicht wird.

10. Pharmazeutische Zusammensetzung für eine Verwendung nach einem der Ansprüche 1 bis 9, **dadurch gekennzeichnet, dass** die pharmazeutische Zusammensetzung mit den Hautverletzungen im Fall einer topischen Verabreichung innerhalb von weniger gleich 60 Sekunden ab dem Kontakt mit der Ursache der Vor-Verbrennung oder der Verbrennung oder im Fall einer Verabreichung mittels Injektion innerhalb von weniger gleich 30 Minuten ab dem Kontakt mit der Ursache der Vor-Verbrennung oder der Verbrennung in Kontakt gebracht wird.

11. Pharmazeutische Zusammensetzung für eine Verwendung nach einem der Ansprüche 1 bis 10, **dadurch gekennzeichnet, dass** die pharmazeutische Zusammensetzung mit den Hautverletzungen im Fall einer topischen Verabreichung innerhalb von weniger gleich 45 Sekunden ab dem Kontakt mit der Ursache der Vor-Verbrennung oder der Verbrennung in Kontakt gebracht wird.

12. Pharmazeutische Zusammensetzung für eine Verwendung nach einem der Ansprüche 1 bis 10, **dadurch gekennzeichnet, dass** die pharmazeutische Zusammensetzung mit den Hautverletzungen im Fall einer Verabreichung mittels Injektion innerhalb von weniger gleich 25 Minuten ab dem Kontakt mit der Ursache der Vor-Verbrennung oder der Verbrennung in Kontakt gebracht wird.

13. Pharmazeutische Zusammensetzung für eine Verwendung nach einem der Ansprüche 1 bis 12, **dadurch gekennzeichnet, dass** die pharmazeutische Zusammensetzung 30 Mal nacheinander innerhalb von 60 Minuten verabreicht wird.

14. Pharmazeutische Zusammensetzung, **dadurch gekennzeichnet, dass** sie mindestens 4 Gew.-%, bezogen auf das Gesamtgewicht der Zusammensetzung, eines Wirkstoffs, der aus Cetearyloctanoat und/oder Capronsäure ausgewählt ist, in Verbindung mit Bienenwachs, einem oder mehreren zusätzlichen Additiven, der bzw. die aus Dexpanthenol und/oder Polydimethylsiloxan (Dimethicon) ausgewählt ist bzw. sind, und gegebenenfalls einem oder mehreren pharmazeutisch unbedenklichen Hilfsstoffen umfasst.

15. Pharmazeutische Zusammensetzung nach Anspruch 14 für deren Verwendung als Arzneimittel.

## Claims

1. A pharmaceutical composition **characterized in that** it comprises at least 4% by weight, relative to the total weight of the composition, of an active ingredient chosen from cetearyl octanoate and/or hexanoic acid, in combination with beeswax and, optionally, one or more pharmaceutically acceptable excipient(s) for use in the treatment of pre-burns or skin burns.

2. The pharmaceutical composition for use as claimed in claim 1, **characterized in that** the active ingredient is cetearyl octanoate.

3. The pharmaceutical composition for use as claimed in claim 1 or 2, **characterized in that** it comprises between 4 and 15% by weight of active ingredient relative to the total weight of the composition.

4. The pharmaceutical composition for use as claimed in one of claims 1 to 3, **characterized in that** it comprises 2 to 5% by weight of beeswax relative to the total weight of the composition.

5. The pharmaceutical composition for use as claimed in one of claims 1 to 4, **characterized in that** the ratio of the active ingredient to the beeswax is between 0,8 and 7,5.

6. The pharmaceutical composition for use as claimed in one of claims 1 to 5, **characterized in that** it comprises one or more additional additive(s) chosen from dexpanthenol and/or polydimethylsiloxane (dimethicone).

7. The pharmaceutical composition for use as claimed in one of claims 1 to 6, **characterized in that** it comprises one or more local anesthetic(s) chosen from: lidocaine, prilocaine, scandicaine, etidocaine, bupivacaine, ropivacaine, levobupivacaine, articaine and mepivacaine.

8. The pharmaceutical composition for use as claimed in one of claims 1 to 7, **characterized in that** it also comprises one or more essential oil(s) or a derivative thereof selected from the group consisting of: *Melaleuca alterniflora* essential oil or a derivative thereof, Tepezcohuite essential oil or a derivative thereof, eucalyptus essential oil or a derivative thereof, thyme essential oil or a derivative thereof, rosemary essential oil or a derivative thereof, lemon essential oil or a derivative thereof, clover essential oil or a derivative thereof, cinnamon essential oil or a derivative thereof, pine essential oil or a derivative thereof, lavender essential oil or a derivative thereof, *Cistus ladaniferus* essential oil or a derivative thereof, St John's Wort essential oil or a derivative thereof and musk rose essential oil or a derivative thereof.

9. The pharmaceutical composition for use as claimed in one of claims 1 to 8, **characterized in that** it is administered topically or by injection.

10. The pharmaceutical composition for use as claimed in one of claims 1 to 9, **characterized in that** the pharmaceutical composition is brought into contact with the cutaneous injuries within a period of less than or equal to 60 seconds starting from the contact with the cause of the pre-burn or burn in the case of a topical administration, or within a period of less than or equal to 30 minutes starting from the contact with the cause of the pre-burn or burn in the case of an administration by injection.

11. The pharmaceutical composition for use as claimed in one of claims 1 to 10, **characterized in that** the pharmaceutical composition is brought into contact with the cutaneous injuries within a period of less than or equal to 45 seconds starting from the contact with the cause of the pre-burn or burn in the case of a topical administration.

12. The pharmaceutical composition for use as claimed in one of claims 1 to 10, **characterized in that** the pharmaceutical composition is brought into contact with the cutaneous injuries within a period of less than or equal to 25 minutes starting from the contact with the cause of the pre-burn or burn in the case of an administration by injection.

13. The pharmaceutical composition for use as claimed in one of 1 to 12, **characterized in that** the pharmaceutical composition is administered 30 times subsequently during 60 minutes.

14. A pharmaceutical composition **characterized in that** it comprises at least 4% by weight, relative to the total weight of the composition, of an active ingredient chosen from cetearyl octanoate and/or hexanoic acid, in combination with beeswax and, optionally, one or more additional additive(s) chosen from dexpanthenol and/or polydimethylsiloxane (dimethicone) and, optionally, one or more pharmaceutically acceptable excipient(s).

15. The pharmaceutical composition according to claim 14 for use in therapy.
